# EUROPEAN PATENT SPECIFICATION

(11) **EP 3 811 083 B1**
(45) Date of publication and mention of the grant of the patent: **14.02.2024**
(21) Application number: 19822034.5
(22) Date of filing: 21.06.2019
(51) Int. Cl.: G01N 33/68

(54) **PROTEIN BIOMARKERS FOR NEPHROPATHY AND APPLICATIONS THEREOF**
PROTEIN-BIOMARKER FÜR NEPHROPATHIE UND ANWENDUNGEN DAVON
BIOMARQUEURS PROTÉIQUES POUR LA NÉPHROPATHIE ET LEURS APPLICATIONS

(30) Priority: 21.06.2018 US 201862688142 P
(43) Date of publication of application: 28.04.2021
(73) Proprietor: China Medical University, Taichung 40402 (TW)
(72) Inventor: CHEN, Chao-Jung, Taichung, Taiwan (TW); TSAI, Fuu-Jen, Taichung, Taiwan (TW)
(74) Representative: Straus, Alexander
(86) International application number: PCT/CN2019/092329
(87) International publication number: WO 2019/242750

(56) References cited:
- CN-A- 104 611 340
- BERNARD A ET AL: "Determination by latex immunoassay of protein 1 in normal and pathological urine", CLINICA CHIMICA ACTA, ELSEVIER BV, AMSTERDAM, NL, vol. 201, no. 3, 30 September 1991 (1991-09-30), pages 231-245, XP024785003, ISSN: 0009-8981, DOI: 10.1016/0009-8981(91)90374-L [retrieved on 1991-09-30]
- KABANDA A ET AL: "LOW MOLECULAR WEIGHT PROTEINURIA IN CHINESE HERBS NEPHROPATHY", KIDNEY INTERNATIONAL, NATURE PUBLISHING GROUP, LONDON, GB, vol. 48, no. 5, 1 November 1995 (1995-11-01), pages 1571-1576, XP000961143, ISSN: 0085-2538, DOI: 10.1038/KI.1995.449
- ASCENSION MARTIN-GRANADO ET AL: "Determination of Clara cell protein urinary elimination as a marker of tubular dysfunction", PEDIATRIC NEPHROLOGY ; JOURNAL OF THE INTERNATIONAL PEDIATRIC NEPHROLOGY ASSOCIATION, SPRINGER, BERLIN, DE, vol. 24, no. 4, 17 January 2009 (2009-01-17), pages 747-752, XP019718033, ISSN: 1432-198X
- CHEN CJ et al.: "Urine proteome analysis by CIS plate-matrix-assisted laser desorption/ ionization time-of-flight mass spectrometry allows noninvasive differential diagnosis and prediction of diabetic nephropathy", PLOS ONE, vol. 13, no. 7, 1 January 2018 (2018-01-01), pages 1-12, XP009508155, ISSN: 1932-6203, DOI: 10.1371/journal.pone.0201073
- MUTTI A et al.: "Exposure to Hydrocarbons and Renal Disease: An Experimental Animal Model", Renal Failure, vol. 21, no. 3-4, 1 January 1999 (1999-01-01), pages 369-385, XP055665181,
- MUTTI A et al.: "Exposure to Hydrocarbons and Renal Disease: An Experimental Animal Model", Renal Failure, vol. 21, no. 3-4, 31 July 1999 (1999-07-31), pages 369-385, XP055665181,
- Monteiro Maria B et al.: "Beta-2-microglobulin (B2M) expression in the urinary sediment correlates with clinical markers of kidney disease in patients with type 1 diabetes", Metabolism, vol. 65, no. 6, 27 February 2016 (2016-02-27), pages 816-824, XP029531773, ISSN: 0026-0495, DOI: 10.1016/j.metabol.2016.02.012

## Description

### RELATED APPLICATION

This application claims the benefit of U.S. provisional application number USSN 62/688,142, filed June 21, 2018 under 35 U.S.C. §119.

### TECHNOLOGY FIELD

The present invention relates to a method for predicting diabetic nephropathy or early progressive renal function decline (ERFD) in a diabetic patient.

### BACKGROUND OF THE INVENTION

Kidney damage, also called nephropathy, can be caused by drug toxicity, inflammation, high blood pressure, and diabetes, for examples. Kidney disease is usually a progressive disease, which means that the damage in the kidneys tends to be permanent and can't be undone. So it is important to identify kidney disease early before the damage is done. Kidney disease can be treated very effectively if it is caught in the early stages. Treatment for chronic kidney disease focuses on slowing the progression of the kidney damage, usually by controlling the underlying cause. Chronic kidney disease can progress to end-stage kidney failure, which is fatal without artificial filtering (dialysis) or a kidney transplant. Specifically, diabetic nephropathy (DN) is one of the most common complications in diabetic patients. Renal disease develops in approximately 20-40% of type 2 diabetic (T2D) patients [1]. In addition, DN is the leading cause of end-stage renal disease (ESRD). Microalbuminuria (urine albumin excretion 30-300 mg/24 h) is the first sign of kidney dysfunction because it can progress to macroalbuminuria (>300 mg/24 h) and subsequently to kidney failure [2,3].

Typically, nephropathy is diagnosed by determining the level of proteinuria (e.g., the level of urine albumin), or by examining the glomerular filtration rate (GFR). Other relevant parameters include systolic blood pressure (SBP), diastolic blood pressure (DBP), fasting blood glucose (FBG), hemoglobin A1c (HbA1c), for example. However, these approaches lack sufficient sensitivity and/or selectivity, especially for detecting early stage nephropathy when no obvious symptoms occur. Although nephropathy can also be detected by renal biopsy, such invasive procedure is not an ideal approach because most patients are reluctant to do so and may thus result in late diagnosis until clinical features are outward or a disease progression has already developed. Renal biopsy also may entail risk for serious bleeding complications. CC16 has been reported as a marker of proximal tubular dysfunction in adult [23] and child patients [24]. In patients with Chinese herbs nephropathy with low molecular weight proteinuria an increase inter alia of B2M and CC16 was found, while total proteinuria and SCr were normal [26].

There is a need to develop a method for detecting nephropathy, especially for general screening, detection at early stage, and in a non-invasive way.

### SUMMARY OF THE INVENTION

The invention is defined in the appended claims.

In this present invention, it is unexpected found that Clara-cell protein (CC16) is specifically and highly expressed in patients with nephropathy compared with control subjects without nephropathy. It is also found that CC16 is highly correlated with development of diabetic nephropathy or early progressive renal function decline (ERFD) in a diabetic patient. Therefore, CC16 can be used as a specific biomarker for diagnosing nephropathy, especially for early detection; and also for predicting diabetic nephropathy or ERFD in a diabetic patient.

The present invention provides a method for predicting diabetic nephropathy or ERFD in a diabetic patient, the method comprising:
(i) providing a biological sample obtained from the diabetic patient; and
(ii) detecting a biomarker in the biological sample to obtain a detection level, comparing the detection level with a reference level for said biomarker to obtain a comparison result, and assessing whether the subject has nephropathy or is at risk of developing nephropathy or ERFD based on the comparison result, wherein the biomarker includes CC16, and an increase in the detection level as compared to the reference level is indicative of a higher risk of developing diabetic nephropathy or ERFD. The biological sample is a urine sample.

If a diabetic patient is determined to have a higher risk of developing diabetic nephropathy or ERFD, the subject may then be subjected to a method for preventing diabetic nephropathy or ERFD.

In some embodiments of the present invention, the detection is carried out by mass spectrometry

In some embodiments of the present invention, one or more additional biomarkers or parameters can be further detected to improve the accuracy of the detection. In certain examples, the biomarker to be detected according to the present invention further includes β2-microglobulin (B2M).

Furthermore, a kit for performing a method as described herein and instructions for using the kit to detect the presence or amount of the biomarker as described herein may be contemplated.

The details of one or more embodiments of the invention are set forth in the description below. Other features or advantages of the present invention will be apparent from the following detailed description of several embodiments, and also from the appended claims.

### BRIEF DESCRIPTION OF THE DRAWINGS

To illustrate the invention, the embodiments are illustrated in the following. However, it should be understood that the invention is not limited to the preferred embodiments shown.

In the drawings:
Fig. 1 shows representative MALDI-TOF mass spectra of urine samples from a healthy individual and patients with WDM-NP, DM-WNP and DM-NP.
Figs. 2A-2B shows excretion of (2A) 11.7 kDa and (2B) 15.8 kDa proteins in urine samples from 39 healthy, 44 WDM-NP, 85 DM-WNP, and 51 DM-NP subjects. The relative intensities are represented as box plots, expressed as the medium with quartile values (25%, 75%). Error bars indicate the minimum and maximum values. * *p* < 0.05, *** *p* ≤ 0.001.
Fig. 3 shows identification of the corresponding peptide of the *m*/*z* 647.91 peak by nanoLC-MS/MS.
Figs. 4A-4B show excretion of (Fig. 4A) β2-microglobulin (B2M) and (Fig. 4B) Clara-cell protein (CC16) in urine samples from 39 healthy, 44 WDM-NP, 85 DM-WNP, and 51 DM-NP subjects. The relative intensities are represented as box plots, expressed as the medium with quartile values (25%, 75%). Error bars indicate the minimum and maximum values. * *p* < 0.05, ** *p* < 0.01, **** p* ≤ 0.001.

### DETAILED DESCRIPTION OF THE EMBODIMENTS

In order to provide a clear and ready understanding of the present invention, certain terms are first defined. Additional definitions are set forth throughout the detailed description. Unless defined otherwise, all technical and scientific terms used herein have the same meanings as is commonly understood by one of skill in the art to which this invention belongs.

As used herein, the articles "a" and "an" refer to one or more than one (*i.e.*, at least one) of the grammatical object of the article. By way of example, "an element" means one element or more than one element.

As used herein, the term "about" or "approximately" refers to a degree of acceptable deviation that will be understood by persons of ordinary skill in the art, which may vary to some extent depending on the context in which it is used. In general, "about" or "approximately" may mean a numeric value having a range of ± 10% around the cited value.

As used herein, the term "comprise" or "comprising" is generally used in the sense of include/including which means permitting the presence of one or more features, ingredients or components. The term "comprise" or "comprising" encompasses the term "consists" or "consisting of."

As used herein, the terms "subject," "individual" and "patient" refer to any mammalian subject for whom diagnosis, prognosis, treatment, or therapy is desired, particularly humans. Other subjects may include cattle, dogs, cats, guinea pigs, rabbits, rats, mice, horses, and so on.

As used herein, the term "diagnosis" as used herein generally includes determination as to whether a subject is likely affected by a given disease, disorder or dysfunction. The skilled persons often make a diagnosis on the basis of one or more diagnostic indicators, *i.e*., a marker, the presence, absence, or amount of which is indicative of the presence or absence of the disease, disorder or dysfunction. It will be understood in the art that diagnosis does not mean determining the presence or absence of a particular disease with 100% accuracy, but rather an increased likelihood of the presence of certain disease in a subject.

As used herein, the term "antibody" means an immunoglobulin protein which is capable of binding an antigen. Antibody as used herein is meant to include the entire antibody as well as any antibody fragments (e.g., F(ab').sub.2, Fab', Fab, Fv) capable of binding the epitope, antigen, or antigenic fragment of interest. Antibodies of the invention are immunoreactive or immunospecific for and therefore specifically and selectively bind to a protein of interest, e.g., CC16 or B2M proteins. Antibodies for the proteins of interest are preferably immunospecific, i.e., not substantially cross-reactive with related materials, although they may recognize their homologs across species. The term "antibody" encompasses all types of antibodies (e.g., monoclonal and polyclonal).

As used herein, the term "treatment" refers to the application or administration of one or more active agents to a subject afflicted with a disorder, a symptom or condition of the disorder, or a progression of the disorder, with the purpose to cure, heal, relieve, alleviate, alter, remedy, ameliorate, improve, or affect the disorder, the symptom or condition of the disorder, the disabilities induced by the disorder, or the progression of the disorder.

As used herein, the term "preventing" refers to preventive or avoidance measures for a disease or symptoms or conditions of a disease, which include but are not limited to applying or administering one or more active agents to a subject who has not yet been diagnosed as a patient suffering from the disease or the symptoms or conditions of the disease but may be susceptible or prone to the disease. The purpose of the preventive measures is to avoid, prevent, or postpone the occurrence of the disease or the symptoms or conditions of the disease.

As used herein, the term "a normal individual" may be used to refer to an individual who is basically in a healthy condition without particular diseases (e.g., nephropathy), and may refer to a single normal/healthy individual or a group of normal/healthy individuals.

As used herein, the term "a control individual" may be used to refer to an individual who does not suffer from a disease of interest (e.g., nephropathy), and may refer to a single control individual or a group of control individuals. In some embodiments, a control individual may refer to normal/healthy individuals. In some embodiments, a control individual may refer to individuals (or diabetic patients) without nephropathy.

As used herein, an "aberrant amount" means an amount of an indicator that is increased as compared to the amount in a subject free from a target disease (e.g., nephropathy) or a reference amount or a control amount. Specifically, for example, an aberrant amount can be higher than a reference amount by more than 5%, 10%, 20%, 30%, 40%, 50%, 60%, 70%, 80%, 90% or 100% or more. A reference amount can refer to the amount measured in control samples (e.g. tissues or cells or any biological samples free from the target disease). In this art, a range of values of normal amounts can be obtained by analyzing detected amounts of a marker in samples from a population of normal individuals using conventional detection and statistic methods.

As used herein, "low expression" and "high expression" for a biomarker as used herein are relative terms that refer to the level of the biomarker found in a sample. In some embodiments, low and high expression can be determined by comparison of the biomarker expression level in a control, non-diseased sample, where low expression can refer to a lower or comparable expression level to the expression level in a control, non-diseased sample, and high expression can refer to a higher expression level to the expression level in a control, non-diseased sample.

As used herein, a biological marker (biomarker) is a characteristic (e.g. a protein, an amino acid, a metabolite, gene or genetic expression) that is objectively measured and evaluated as an indicator of normal or abnormal biologic processes, diseases, pathogenic processes, or responses to treatment or therapeutic interventions. Biomarkers can include presence or absence of characteristics or patterns or collections of the characteristics which are indicative of particular biological processes. The biomarker measurement can increase or decrease to indicate a certain biological event or process. A marker is primarily used for diagnostic and prognostic purposes. However, it may be used for therapeutic, monitoring, drug screening and other purposes described herein, including evaluation the effectiveness of a therapeutic.

As used herein, a biological sample is a urine sample.

As used herein, the term "physiological parameter", as used herein, refers generally to any parameter that may be monitored to determine one or more quantitative physiological levels and/or activities associated with the patient. Examples of the physiological parameter include but are not limited to age, gender, systolic blood pressure (SBP), diastolic blood pressure (DBP), fasting blood glucose (FBG), hemoglobin A1c (HbA1c), diabetes duration, creatinine, estimated glomerular filtration rate (eGFR), albuminuria, urine albumin to creatinine ratio (ACR), and any combination thereof. In some certain embodiments, the physiological parameter includes fasting blood glucose (FBG) and/or diastolic blood pressure (DBP).

As used herein, the term "nephropathy" refers to a physiological condition wherein damage of the kidney occurs, which specifically disrupts its ability to properly regulate solute concentrations in the blood and urine. A nephropathy can be characterized by one or more pathological changes: glomerular size, fibrosis of the tufts, fibrosis of Bowman's capsule, dilatation, narrowing of capillaries, thickening of basement membranes, increased cellularity (mesangial or endothelial), infiltration by leukocytes, capillary thrombi, tubules-atrophy, necrosis, vacuolar and hyaline droplet changes, basement membrane thickening, dilatation, inflammatory cells and casts in the lumen, interstitium-fibrosis, edema, acute and chronic leukocyte infiltration, arterioles-fibrosis, thrombosis, hyaline change and narrowing. Generally, in the early stage of nephropathy, the kidneys are still able to work well to filter out waste from the blood; in the middle stage, the kidneys may have to work harder to get rid of waste; and in the late stage, the kidneys may stop working. Typically and conventionally, nephropathy can be assessed by urinary protein concentration. The early clinical feature for nephropathy can be a low but abnormal concentration of albumin (albumin excretion rate, AER: 30-300 mg/24h; or albumin to creatinine ratio, ACR: 30-300 mg/g) in urine, called microalbuminuria, and this patient has initial nephropathy (incipient nephropathy); without proper treatment, such patients will develop persistent microalbuminuria and turn into severe nephropathy (overt nephropathy), also called macroalbuminuria (AER > 300 mg/24 hours or ACR>300mg/g), and finally progress to end stage renal disease (ERSD). Estimated glomerular filtration rate (eGFR) can also be used as an indicator for nephropathy. Chronic kidney disease (CKD) can be defined by having eGFR below 60 ml/min in patients with or without proteinuria for more than 3 months; or having proteinuria for more than 3 months in spite of low or high level of eGFR. Nephropathy can also be assessed based on, for example, serum creatinine concentration, urinary protein concentration, urinary protein to creatinine ratio or through the use of tracer compounds such as phthalates.

In some embodiments, CKD can be deemed to include five (5) stages of kidney damage, from very mild damage in stage 1 to complete kidney failure in stage 5. See Table A.

**Table A. different stages of CKD.**

| CKD Stage | Features |
|---|---|
| Stage 1 | eGFR is greater than (and equal to) 90 ml/min. |
| | Kidneys are still working well. |
| | Usually, no symptoms are found. |
| | Other signs of kidney damages (e.g. proteinuria) are observed. |
| Stage 2 | eGFR is between 60 and 89 ml/min. |
| | Kidneys are still working well. |
| | Usually, no symptoms are found. |
| | Other signs of kidney damages (e.g. proteinuria) are observed. |
| Stage 3 | eGFR is between 30 and 59 ml/min. |
| | Kidneys are moderately damaged and are not working as well as they should. |
| | Most patients still do not have any symptom, but sometimes, common symptoms are found e.g. swelling in hands and feet, back pain and urinating more or less than normal. |
| Stage 4 | eGFR is between 15 and 29 ml/min. |
| | Kidneys are moderately or severely damaged and are not working as well as they should. |
| | More patients have symptoms, e.g. swelling in hands and feet, back pain and urinating more or less than normal. |
| Stage 5 | eGFR is less than 15. |
| | Kidneys are severely damaged and very close to failure or have completely failed. |
| | The patients have more severe symptoms e.g. itching, nausea, vomiting, trouble breathing, due to renal failure and accumulation of toxins and wastes in blood. |

Specifically, an early stage of CKD as described herein can include stage 1 and stage 2 as shown above that such patients may have relatively higher (normal) eGFR but have at least one sign of kidney damages e.g. microalbumin.

As used herein, the term "diabetic nephropathy" refers to renal diseases resulting from diabetes. In certain embodiments, the diabetes is type 2 diabetes. Many diabetic patients have experienced early progressive renal function decline (ERFD) before microalbuminuria onset, although they may still have normal kidney function. Once the process of decline begins, without proper treatment, it progresses and could lead to impaired kidney function. Specifically, ERFD can be determined when there is an anural loss of more than 3.3 mL/min per 1.73 m² decline in eGFR.

The present disclosure is based (at least in part) on the identification of CC16 as a novel reliable nephropathy biomarker. As demonstrated in some examples below, an increased level of CC16 is found in the urine samples of individuals suffering from nephropathy. Thus, the nephropathy detection method described herein can be used to identify whether an individual has, is suspected of having, or is at the risk of developing nephropathy. The detection method described herein can be applied to any subject, especially as an initial, regular and routine (or early-stage) screening method to identify those with nephropathy or at the risk for progressing nephropathy. As further demonstrated in other examples below, the presence of CC16 in diabetic patients is associated with later development of ERFD. Thus, when applying in diabetic patients, the detection method described herein can be used to predict the risk to develop ERFD or diabetic nephropathy.

In some embodiments, B2M is further detected to increase accuracy of the detection.

As used herein, Clara cell protein (CC16) is a 15.8-kDa homodimeric protein which is secreted in large amounts in airways by the non-ciliated bronchiolar Clara cells. CC16 has been shown to modulate the production and/or the activity of various mediators of the inflammatory response including PLA2, interferon-gamma and tumour necrosis factor-alpha (Clinical & Experimental Allergy 30(4):469-75 · May 2000). β2-microglobulin (B2M) is a subunit of the major histocompatibility complex (MHC) class I molecule. The amino acid sequences of these protein biomarkers and are well known in the art, for example, CC16: P11684, and B2M: P61769.

The presence and amount of the biomarkers as described herein in a biological sample can be determined by routine technology. In some embodiments, the presence and/or amount of the biomarkers as described herein can be determined by mass spectrometry, which allows direct measurements of the analytes with high sensitivity and reproducibility. A number of mass spectrometric methods are available. Examples of mass spectrometry include, but are not limited to, matrix-assisted laser desorption ionization/time of flight (MALDI-TOF), surface-enhanced laser desorption ionisation/time of flight (SELDI-TOF), liquid chromatography-mass spectrometry (LC-MS), liquid chromatography tandem mass spectrometry (LC-MS-MS), and electrospray ionization mass spectrometry (ESI-MS). One certain example of this approach is tandem mass spectrometry (MS/MS), which involves multiple steps of mass selection or analysis, usually separated by some form of fragmentation.

In other embodiments, the presence and/or amount of a biomarker can be determined by an immunoassay. Examples of the immunoassays include, but are not limited to, Western blot, enzyme-linked immunosorbent assay (ELISA), radioimmunoassay (RIA), radioimmunoprecipitation assay (RIPA), immunofluorescence assay (IFA), ELFA (enzyme-linked fluorescent immunoassay), electrochemiluminescence (ECL), and Capillary gel electrophoresis (CGE). In some examples, the presence and/or level of a biomarker can be determined using an agent specifically recognizes said biomarker, such as an antibody that specifically binds to the biomarker.

Antibodies as used herein may be polyclonal or monoclonal. Polyclonal antibodies directed against a particular protein are prepared by injection of a suitable laboratory animal with an effective amount of the peptide or antigenic component, collecting serum from the animal, and isolating specific sera by any of the known immunoadsorbent techniques. Animals which can readily be used for producing polyclonal antibodies as used in the invention include chickens, mice, rabbits, rats, goats, horses and the like.

For the performing of the method described herein, the detection or the measurement of the amount of a biomarker as described herein in the biological sample taken from an individual in need thereof (e.g., a human patient who does not have any symptoms of nephropathy, or a human patient suspected of having, or at risk of having nephropathy) is carried out by any method known in the art, such as those described herein, e.g. mass spectrometry The biological sample is a urine sample.

In some embodiments, the amount of a biomarker in the sample derived from the candidate individual can be compared to a standard value. A higher amount of the biomarker as described herein can indicate a positive result i.e. that the individual has nephropathy or be at risk of developing nephropathy, or the individual has a higher risk of developing diabetic nephropathy or ERFD when he/she is a diabetic patient. The standard value represents the amount of a biomarker as described herein in the control sample. The control sample can be taken from an individual that does not have nephropathy. Additionally, the control sample can be a mixture of samples taken from a group of such individuals. Alternatively, the control individuals are matched to the candidate individual in, for example, age, gender, and/or ethnic background. Preferably, the control sample and the biological sample of the candidate individual are samples of the same species.

In some certain examples, the level of the marker(s) in a control sample is non-detectable in a control sample (i.e. the reference value being 0) using a routine assay e.g. mass spectrometry and immunoassays, and the presence of the marker as detected (detectable marker) in a biological sample from a subject using the same assay can indicate a positive result.

In some embodiments, CC16 is detected as a first biomarker according to the present invention. A higher level of the first biomarker as compared to a (first) control level of said biomarker can indicate a first positive result. In some additional embodiments, B2M is further detected as a second biomarker according to the present invention. A higher level of the second biomarker as compared to a (second) control level of said biomarker can indicate a second positive result, with increased accuracy. In still further embodiments, one or more physiological parameter can be additionally measured. For example, such physiological parameter may be selected from the group consisting of estimated glomerular filtration rate (eGFR), albuminuria, urine albumin to creatinine ratio (ACR), and any combination thereof.

When an individual, such as a human patient, is diagnosed as having, suspected of having, or at risk of having nephropathy, the individual may undergo further testing (e.g., routine physical testing, including surgical biopsy or imaging methods, such as X-ray imaging, magnetic resonance imaging (MRI), or ultrasound) to confirm the occurrence of the disease and/or to determine the stage and type of nephropathy.

The methods described herein can further be followed by treating the nephropathy patient to at least relieve symptoms associated with the disease. The treatment can be conducted by administration of conventional medicaments for nephropathy. Examples of such medicaments include but are not limited to (i) drugs for reducing albuminuria such as a phosphodiesterase inhibitor e.g. dipyridamole and pentoxifylline; (ii) anti-hypertensive drugs such as an angiotensin converting enzyme (ACE) inhibitor e.g. imidapril and an angiotension receptor blocker (ARB) e.g. losartan; (iii) phosphate binders such as sevelamer carbonate, lanthanum carbonate and Al (OH)₃ hexitol complex; (iv) calcium supplements such as calcium carbonate, calcium citrate and vitamin D; (v) anti-anemia drugs such as erythropoietin (EPO) and iron supplements; (vi) drugs for lowering blood fat such as statins e.g. simvastatin, pravastatin and atorvastatin; (vii) drugs for reducing uric acid such as allopurinol, febuxostat and benzbromarone; (viii) others, for example, corticosteroids such as prednisolone, non-steriodal anti-inflammatory drugs (NSAIDs) and N-acetylcysteine (for preventing contrast-induced nephropathy, CIN). The medicines can be administered in an effective amount to a subject in need. The treatment of nephropathy may also comprise food therapy with a low protein and/or a low salt diet.

As used herein, "effective amount" refers to the amount of each active substance that can be administered to the individual, either alone or in combination with one or more other active substances, to confer therapeutic effect on the individual. The effective amount may vary and must be determined by those skilled in the art, depending on the specific circumstances at the time of administration, the severity of the condition, respective parameters of patients, including age, gender, age, weight, height, physical condition, treatment schedule, the nature of the parallel therapy (if any), the specific route of administration, and other possible factors judged by the knowledge and profession of medical personnel. Such factors are well known to those of ordinary skill in the art and can be introduced without further routine experimentation.

A kit or composition for performing the method is also contemplated, which comprises a reagent (e.g., an antibody, or a labeling reagent) that specifically recognizes a biomarker as described herein. The kit may further comprise instructions for using the kit to detect the presence or amount of the biomarker described herein, thereby detecting nephropathy in a subject in need thereof, or for predicting diabetic nephropathy or ERFD in a diabetic patient. The components including the detection reagents as described herein can be packaged together in the form of a kit. For example, the detection reagents can be packaged in separate containers, e.g., antibodies (either bound to a solid matrix or packaged separately with reagents for binding them to the matrix), a control reagent (positive and/or negative), and/or a detectable label, and the instructions (e.g., written, tape, VCR, CD-ROM, *etc*.) for performing the assay can also be included in the kit. The assay format of the kit can be a chip or an ELISA, for example. Further provided is use of such reagent for performing a method described herein. Such reagent includes a reagent that specifically recognizes the biomarker. In some embodiments, such reagent includes (i) a molecule that specifically recognizes CC16, optionally (ii) a molecule that specifically recognizes B2M, or (iii) a combination of (i) and (ii). The reagent may be mixed with a carrier e.g. a pharmaceutically acceptable carrier to form a composition for the detection or diagnosis purpose. Examples of such carrier include injectable saline, injectable distilled water, an injectable buffer solution and the like.

Without further elaboration, it is believed that those skilled in the art will be able to apply the invention to its fullest extent based on the above description. The following specific examples are, therefore, intended to be illustrative, and are not intended to limit the applicable scope of the invention in any way. The scope of the invention shall be defined by the appended claims.

### Examples

In this study, a C₁₈ plate and matrix-assisted laser desorption/ionization time-of-flight mass spectrometry (MALDI-TOF-MS) were used to compare the urinary protein profiles of 238 subjects from the following 4 groups: patients with type 2 diabetic (T2D) with microalbuminuria, patients with DM without micro- or macroalbuminuria, patients with micro- or macroalbuminuria due to nondiabetic disease, and healthy controls. β2-microglobulin (B2M) and Clara-cell protein (CC16) were found to be highly released in the urine of patients with proteinuria due to nondiabetic or diabetic diseases. In differentiating nephropathy from healthy subject, the B2M and CC16 markers have a combined sensitivity and specificity of 77.3% and 91.8%, respectively. In distinguishing T2D with microalbuminuria from T2D patients, the combined markers have sensitivity and specificity of 66% and 73%, respectively. The predictive ability of B2M and CC16 for early renal functional decline (ERFD) was validated in 125 T2D patients with a follow-up times. The odds ratio (OR) of combined B2M and CC16 markers for developing ERFD was 7.59 (95% CI: 1.97-29.24). The detection of B2M and CC16 with the C₁₈ plate-MALDI-TOF MS approach could be an attractive and practical assay for rapid diagnosis of nephropathy in nondiabetic/diabetic patients and as a predictor of ERFD among T2D patients who had not manifested significant kidney disease at baseline.

Abbreviations: DM=diabetes mellitus; DN=diabetic nephropathy; WDM-NP=patients with micro- or macroalbuminuria due to nondiabetic disease; DM-WNP=patients with type 2 diabetes mellitus without micro- or macroalbuminuria; DM-NP=patients with type 2 diabetes mellitus with microalbuminuria; MALDI-TOF-MS=matrix-assisted laser desorption/ionization time-of-flight mass spectrometry; SELDI-TOF-MS= surface-enhanced laser desorption/ionization time-of-flight mass spectrometry; SA=Sinapic acid; CC16= Clara-cell protein; B2M=β2-microglobulin.

### 1. Materials and Methods

### 1.1 Chemicals

Polydimethylsiloxane (PDMS) prepolymer was purchased from Dow Corning (Sylgard 184Midland, MI, USA). Acetonitrile (ACN) and trifluoroacetic acid (TFA) were purchased from J.T. Baker (Phillipsburg, NJ, USA). Dithiothreitol (DTT), iodoacetamide (IAA), and formic acid (FA) were purchased from Sigma-Aldrich(St Louis, MO, USA). Octadecyl-coated silica particles (C₁₈, 3 µm, 100 Å, Develosil) were purchased from Nomura Chemical Co., Ltd (Seto, Japan). Sinapic acid (SA) was purchased from Bruker Daltonics (Germany). Trypsin (modified, sequencing grade) was obtained from Promega (Madison, WI, USA). Urea, was purchased from Bio Basic Inc. (Toronto, Canada).

### 1.2 Study population and samples

A cross-sectional study design was used for the discovery and validation of protein markers by C18 plate/MALDI-TOF MS. The study protocol, including sample collection, preparation, and analysis, was approved by the local ethics committee of the China Medical University Hospital, Taichung, Taiwan, and performed according to the principles of the Declaration of Helsinki. All subjects (n = 238) had given their informed consent before the study. The following 4 groups were defined according to clinical course and urinary albumin excretion levels: patients with DM with microalbuminuria (DM-NP; n = 53, 30 < albumin-to-creatinine (ACR) ratio < 300 mg/g), patients with DM without micro- or macroalbuminuria (DM-WNP; n = 87, ACR < 30 mg/g), patients with micro- or macroalbuminuria due to nondiabetic disease (WDM-NP; n = 48, 30 mg/g < ACR), and healthy controls (n = 50; ACR < 30 mg/g). The clinical characteristics of the 4 groups are shown in Table 1.

**Table 1. Clinical and biochemical parameters for the healthy, WDM-NP, DM-WNP, and DM-NP subjects.**

| | **Unit** | **Healthy (n=50)** | **WDM-NP (n=48)** | **DM-WNP (n=87)** | **DM-NP (n=53)** |
|---|---|---|---|---|---|
| **Gender (M/F)** | None | 23/27 | 19/29 | 47/40 | 27/26 |
| **Age** | years | 51.6 (7.6) | 64.3 (15.3) | 61.1 (11.0) | 65.5 (11.5) |
| **BMI** | kg/m² | 23.5 (3.5) | 25.2 (5.3) | 25.0 (3.2) | 26.2 (4.0) |
| **HbA1c** | % | 5.5 (0.4) | 5.8 (0.4) | 6.6 (0.5) | 6.9 (0.6) |
| **Creatinine** | mg dL⁻¹ | 1.0 (0.2) | 1.2 (1.4) | 0.80 (0.20) | 0.80 (0.20) |
| **eGFR** | ml/min | 76.0 (10.7) | 67.1 (28.0) | 92.8 (22.7) | 88.6 (24.0) |
| **Albuminuria** | mg dL⁻¹ | 0.7 (0.5) | 534.6 (980.0) | 7.1(8.1) | 85.9(103.1) |
| **Urine creatinine** | mg dL⁻¹ | 118.7(66.2) | 141.9 (228.9) | 90.3 (59.3) | 78.7 (53.3) |
| **Urine ACR** | mg/g | 6.3 (4.0) | 961.1 (1911.5) | 8.1 (6.9) | 106.1 (70.4) |

| | | | | | |
|---|---|---|---|---|---|
| Values are expressed as the mean ± standard deviation. WDM-NP group, patients with micro- or macroalbuminuria due to nondiabetic disease; DM-WNP group, patients with diabetes mellitus (DM) without micro- or macroalbuminuria; DM-NP, patients with DM with microalbuminuria; BMI, body mass index; eGFR, estimated glomerular filtration rate; ACR, albumin-to-creatinine ratio. | | | | | |

### 1.3 Study population for follow-up verification

A total of 125 T2D subjects, including 56 had ERFD (case) and 69 did not have ERFD (control) were included in this nested case-control study. All patients had normal renal function (estimated glomerular filtration rate [eGFR] > 60 mL/min per 1.73 m² and an ACR < 300 mg/g) at the time of enrollment. Among these participants, 56 had ERFD, and 69 did not have ERFD during follow-up. ERFD is defined as having more than 3.3 mL/min per 1.73 m² decline in eGFR per year [12]. The Modified Diet in Renal Disease (MDRD) equation [13] was used to estimate GFR.

### 1.4 Urine sample preparation for protein profiling

Midstream urine was collected in a 15-mL centrifuge tube for protein sampling. To reduce the protein degradation effect, 500 µL of a protease inhibitor cocktail solution (1 protease inhibitor tablet dissolved in 10 mL of double-distilled water (ddH₂O)) was added to 10 mL of each collected urine sample. The urine samples were centrifuged for 20 min at 3000 g and 4 °C. After elimination of the precipitate, the supernatant was collected for use immediately or stored at -80 °C.

### 1.5 Protein desalting by C₁₈ plate and protein profiling by MALDI-TOF MS

The C₁₈ plates were fabricated according to our previous study [11]. The C₁₈ spots were first washed with a 100% MeOH solution to remove contaminants and nonspecifically adsorbed compounds. The urine sample (20 µL) was directly loaded onto the C₁₈ spots and incubated for 10 min or until they had dried. The spots were then washed with ddHzO to remove salts. The desalted proteins were eluted from the C₁₈ plate using 3 µL of an 80% ACN/0.1% TFA solution. For MALDI-TOF MS analysis, the eluted proteins were mixed with 2 µL of SA solution (saturated SA in 30% ACN/0.1% TFA) on a MALDI-target. After SA/protein co-crystallization, the MALDI-target was analyzed with a MALDI-TOF/TOF MS system (Ultraflex III TOF/TOF; Bruker Daltonics) equipped with a Smartbeam laser system, using the linear mode.

### 1.6 Purification of protein marker peaks

A liquid chromatography (LC) pumping system (Ultimate 3000; Dionex) equipped with an LC column (XBridge Protein BEH C₄ column, 300 Å, 3.5 µm, 2.1 mm × 150 mm; Waters) was used for purifying the protein. The mobile phases were solvent A (5% ACN and 0.1% FA) and solvent B (100% ACN and 0.1% FA). Gradient elution at a flow rate of 250 µL/min was set as follows: 1% B for 1.5 min, 1% to 30% B over 1 min, 30% to 80% B over 16 min, 80% B for 2 min, and 80% B to 1% B over 5 min. The eluents were monitored with a UV detector (VWD-3400 RS; Dionex) at the wavelengths of 220 and 280 nm. The eluents were collected at 60-s intervals. Each fraction was analyzed by MALDI-TOF MS to confirm the successful purification of the marker peak at *m*/*z* ~15860. The purified protein subfraction with the marker peak at *m*/*z* 15860 and its neighboring subfractions (as control subfractions) were dried in a centrifugal concentrator (miVac Duo Concentrator; Genevac, NY, USA) and then subjected to in-solution digestion and nanoLC-MS/MS analysis for identification.

### 1.7 In-solution digestion

The purified protein marker peak at *m*/*z* 15800 was re-dissolved in 4 M urea and reduced with 10 mM DTT for 45 min at 37 °C. Then, 55 mM IAA was added and the mixture was incubated for 60 min in the dark at 25 °C. Ammonium bicarbonate buffer (10 mM) was added to the protein solution to reduce the urea concentration to below 1 M. Trypsin was then added to the protein solution at an enzyme-to-substrate ratio of 1:25 (w/w) for 16 h at 37 °C. The peptide solution was desalted with C₁₈ Z-tips, dried in a centrifugal concentrator, and then reconstituted with 10 µL of 0.1% FA for nanoLC-MS/MS analysis.

### 1.8 NanoLC-MS/MS analysis

NanoLC-MS/MS was performed with a nanoflow ultra-performance liquid chromatography system (UltiMate 3000 RSLCnano system; Dionex) coupled to a hybrid quadrupole time-of-flight (Q-TOF) mass spectrometer (maXis Impact; Bruker). After sample loading, the peptides were eluted frim a trap column into an analytical column (Acclaim PepMap C₁₈, 2 µm, 100 Å, 75 µm × 250 mm; Thermo Scientific) coupled to a nano-electrospray ionization source on the Q-TOF mass spectrometer. A gradient elution of 8% ACN (0.1% FA) to 40% ACN (0.1% FA) over 36 min was used at a flow rate of 300 nL/min for tryptic peptide separation. Eight precursors of charge +2, +3, and +4 from each TOF MS scan were dynamically selected and isolated for MS/MS fragment ion scanning. The MS and MS/MS accumulation were set at 1 and 10 Hz, respectively.

### 1.9 Protein database search

The spectra acquired by nanoLC-MS/MS were converted into xml files using DataAnalysis (version 4.1; Bruker) and searched against the Swissprot (release 51.0) database using MASCOT (version 2.2.07). The MASCOT search parameters for precursor ion and fragment ion tolerance were 80 ppm and 0.07 Da, respectively. The following search parameters were selected: Taxonomy, Human; missed cleavages, 1; enzyme, trypsin; fixed modifications, carbamidomethyl (C); and variable modifications, oxidation (M) and deamidation (NQ). Peptides were considered as "identified" if their individual MASCOT ion score was higher than 25 (*p* < 0.01).

### 1.10 ELISA measurement of B2M and Clara-cell protein in urine

The urine B2M and Clara-cell protein (CC16) concentrations were measured by ELISA using commercial kits (Cloud-Clone Corp.) according to the manufacturer's instructions. All samples were processed using the same equipment and by the same laboratory technician, who was blinded to all clinical data. The Mann-Whitney test was used to compare differences in the medium values, which were expressed as the medium with quartile values (25%, 75%).

### 1.11 Statistical analysis

Continuous data were presented as means and standard deviations or medians and interquartile ranges, and categorical data were presented as proportions. Two independent sample T-test was used for comparisons of means of continuous variables, and chi-squared test was used for comparisons of the frequencies of categorical variables between groups. The association between potential urinary biomarkers and ERFD was estimated using logistic regression model, and odd ratios (ORs) and 95% confidence intervals (CIs) were calculated. The receiver operating characteristic (ROC) curve was constructed to determine the sensitivity and specificity of the potential biomarker. Statistical analyses were conducted using SigmaPlot 11.1 (Systat Software Inc., CA, USA) and SPSS statistical software, 22.0 (IBM Corp., NY, USA). The *p* values of less than 0.05 (two-sided) were considered significant

### 2. Results

### 2.1 Urine Sample preparation by C₁₈ plate

A high salt content in urine could interfere with MALDI crystallization and result in poor MS signals. To evaluate the salt effect on the urinary protein profiling, 20 µL of urine was directly applied to MALDI-TOF MS analysis without desalting. To avoid the salt interference effect, we used a hydrophobic C₁₈ plate to remove salts but retain proteins in the urine samples. After applying the desalted urine sample to the C₁₈ plate, the protein signals were greatly improved.

To evaluate the minimum protein amount required for acquiring a protein profile in this study, different urine protein amounts were tested. Similar protein profiles were obtained when 0.6-2.38 µg amounts were applied. The protein concentrations (expressed as the medium with quartile values (25%, 75%)) measured by Bradford protein assay in urine samples from the healthy, WDM-NP, DM-WNP, and DM-NP groups were 0.06 µg/µL (0.03-0.09 µg/µL), 0.34 µg/µL (0.13-1.11 µg/µL), 0.05 µg/µL (0.03-0.08 µg/µL), and 0.13 µg/µL (0.10-0.19 µg/µL), respectively. Therefore, the protein amount in 20 µL of urine sample was sufficient to give an informational protein profile in this study.

### 2.2 Protein excretion patterns in normal and pathological urines

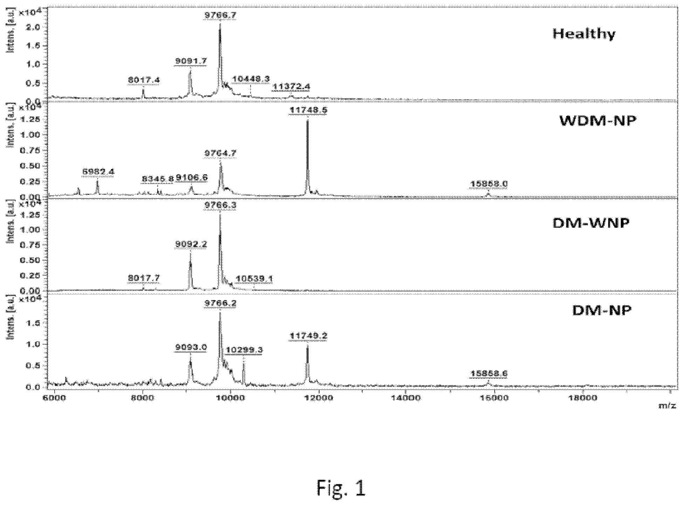

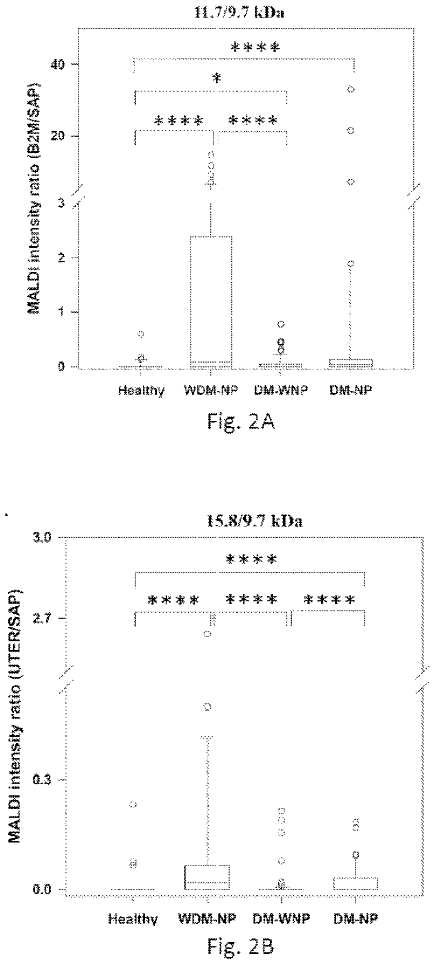

Desalted urinary protein samples from 87 DM-WNP patients, 53 DM-NP patients, 48 WDM-NP patients, and 50 healthy controls were analyzed by MALDI-TOF MS. The representative MALDI-TOF mass spectra from healthy and WDM-NP subjects are shown in Fig. 1. The prominent peaks of *m*/*z* 11732±2 (or oxidized form *m*/*z* 11748± 2) and *m*/*z* 15840±3(or oxidized form m/z ~15856±3) were found to be highly expressed in WDM-NP and DM-NP subjects; their representative pseudo-gel and spectrum were also performed (data not shown). The peak of 9.7 kDa (saposine B) was used as the internal standard to evaluate the diagnostic value of the two protein marker peaks of 11.7 kDa and 15.8 kDa. As shown in Fig. 2A, the peak ratio of 11.7/9.7 kDa is significantly higher in WDM-NP and DM-NP than in healthy group (p<0.001). The peak ratio of 15.8/9.7 kDa is significantly higher in WDM-NP and DM-NP than in healthy (p<0.001) and DM-WNP groups (p<0.001) (Fig. 2B).

To distinguish WDM-NP patients from healthy subjects, the area under the curve (AUC) of the ROC plots was investigated. The AUC was 0.75 for the 11.7 kDa peak and 0.74 for the 15.8 kDa peak. Because these two peaks can be simultaneously examined in a single MALDI-TOF mass spectrum, both can be used as diagnostic markers. These 2 peaks gave a sensitivity and specificity of 77.3% and 91.8%, respectively, with an improved AUC of 0.8 and therefore could be used as markers to discriminate between WDM-NP (nephropathy) and healthy subjects. For the differentiation of DM-NP (diabetic nephropathy) from DM-WNP patients, the AUC was 0.6 for the 11.7 kDa peak and 0.67 for the 15.8 kDa peak. The combined markers of these two peaks in this case had a sensitivity and specificity of 66 % and 73 % with the AUC of 0.62.

### 2.3 Purification and identification of differentially expressed proteins

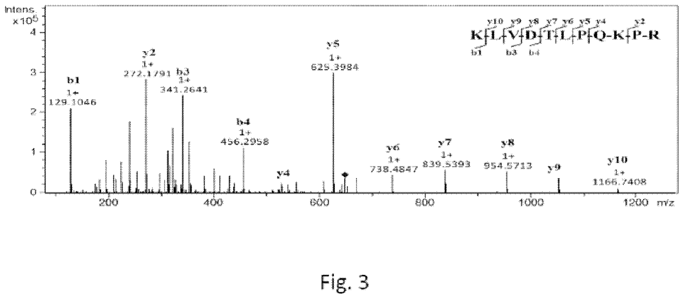

The 11.7 kDa peak has been reported to be B2M [14] and was also identified in our previous study [15]. To confirm the identity of the 15.8 kDa peak, the urine samples were fractionated by C₄ reversed-phase chromatography as described in the Materials and Methods section. The LC-UV chromatogram of urinary proteins from a DM-NP subject was performed (data not shown), and the 15.8 kDa peak was purified from subfraction 10,which was identified as CC16 (Mascot identification score of 88) on the basis of MS/MS sequencing of the doubly charged tryptic peptide peak of *m*/*z* 647.91, showing a complete y- and b-ion series corresponding to the sequence KLVDTLPQKPR (Fig.3).

### 2.4 ELISA evaluation of B2M and CC16

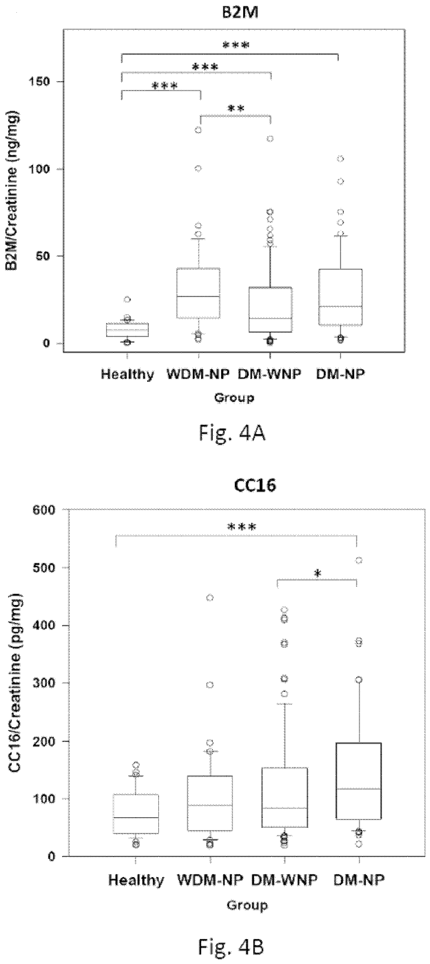

Because ELISA is often used in the clinical laboratory to quantify protein marker abundance for diagnosis, the 11.7 kDa B2M and 15.8 kDa CC16 were subjected to ELISA to evaluate the relative abundance of the two markers in urine. (Fig. 4) B2M expression was significantly higher in the WDM-NP, DM-NP, and DM-WNP groups than in the healthy group (p < 0.001). However, CC16 expression was significantly high in the DM-NP group only relative to the DM-WNP (*p* < 0.05) and healthy (*p* < 0.001) groups. To distinguish WDM-NP patients from healthy subjects, the AUC of the ROC plot was evaluated and found to be 0.87 for B2M and 0.67 for CC16, and the combined AUC for B2M and CC16 was 0.74. In the case of distinguishing DM-NP from DM-WNP, the AUC was 0.59 for B2M and 0.60 for CC16, and the combined AUC was 0.60.

### 2.5 Validation of the protein markers in T2D patients who had developed to ERFD

A nested case control study design was used for investigating the prediction ability of B2M and CC16 in the development of nephropathy in T2D patients. The T2D subjects with (n = 56) and without (n = 69) ERFD as primary end point had similarly demographic (age and gender), DM duration, BMI, SBP, DBP, HbA1c, creatinine, and ACR at the baseline examination. Patients with ERFD had higher eGFR than those without ERFD (means ± standard deviations: 114.13 ± 25.86 versus 98.86 ± 23.36 [p value = 0 .001]). ACR is not a significant marker in baseline for predicting ERFD (p value for chi-square test was 0.196 (Table 2)). For the two potential urinary markers, the 16 of 56 (28.6%) T2D patients with ERFD and the 9 of 69 (13.0%) T2D patients without ERFD had detectable CC16 marker (CC16/SAP ratio > 0) at baseline (p value for chi-square test was 0.031 (Table 2)). No significant difference between two groups for presence of B2M was observed (39.3% vs. 31.9% for ERFD and non ERFD group, *p* = 0.389). The data showed that the presence of CC16 at baseline is associated with the later development of ERFD.

**Table 2. Demographic and clinical characteristics stratified by decline in eGFR/year ≧3.3% (ERFD)**

| | | | Stable (N= 69) | Rapid decline (N = 56) | P value |
|---|---|---|---|---|---|
| | Age | | 54.84 (7.98) | 56.07 (8.16) | 0.399 |
| | Male% | | 40 (58.0%) | 30 (54.5%) | 0.702 |
| | Follow up duration | | 3.81 (1.88) | 3.33 (1.60) | 0.124 |
| | DM duration | | 8.80 (7.40) | 8.30 (5.88) | 0.759 |

| **At haptoglobin measurement** | | | | | |
|---|---|---|---|---|---|
| | HbAlc | | 7.94 (1.72) | 7.92 (1.94) | 0.927 |
| | Urine Creatinine (mg dL⁻¹) | | 173.62 (136.44) | 145.60 (75.35) | 0.150 |
| | eGFR | | 98.86 (23.36) | 114.13 (25.86) | 0.001 |
| | BMI | | 25.63 (3.77) | 26.10 (4.11) | 0.510 |
| | SBP | | 122.12 (18.51) | 127.60 (18.31) | 0.102 |
| | DBP | | 71.71 (11.34) | 72.62 (10.65) | 0.650 |

| **Biomarker concentrations** | | | | | |
|---|---|---|---|---|---|
| | ACR | | 22.94 (31.25) | 33.45 (52.89) | 0.196 |
| | B2M/SAP_MALDI ratio (>0 %) | | 22 (31.9%) | 22 (39.3%) | 0.389 |
| | CC16/SAP MALDI ratio (>0 %) | | 9 (13.0%) | 16 (28.6%) | 0.031 |

| **Combine B2M/SAP**_**MALDI and CC16/SAP MALDI ratio** | | | | | |
|---|---|---|---|---|---|
| | | Group1 | 43 (62.3) | 28 (50.0) | 0.153 |
| | | Group2 | 21 (30.4) | 18 (32.1) | |
| | | Group3 | 5 (7.2) | 10 (17.9) | |

| | | | | | |
|---|---|---|---|---|---|
| Abbreviation: ACR, albumin to creatinine ratio; SBP, systolic blood pressure; DBP, diastolic blood pressure; eGFR, estimated glomerular filtration rate; BMI, body mass index; ERFD, early renal functional decline; DM, diabetes mellitus. Group1: B2M/SAP_MALDI ratio = 0 and CC16/SAP MALDI ratio = 0 Group2: B2M/SAP_MALDI ratio = 0 and CC16/SAP MALDI ratio > 0, B2M/SAP_MALDI ratio > 0 and CC16/SAP MALDI ratio = 0 Group3: B2M/SAP_MALDI ratio > 0 and CC16/SAP MALDI ratio > 0 | | | | | |

Logistic regression was used to further examine the effect of potential biomarkers, B2M and CC16, independently or combined on ERFD (Table 3). When comparing individuals having marker with those without marker, the OR for ERFD was 2.01 (95% CI: 0.90-4.52) for B2M marker and 4.87 (95% CI: 1.77-13.44) for CC16 marker, respectively after adjusting for follow-up time. Furthermore, there was a significant additive effect of increasing the ERFD risk with combined CC16 and B2M (the *p* value of interaction term = 0.003). The OR for ERFD was 7.59 (95% CI: 1.97-29.24) when comparing individuals having both markers with those without any markers.

**Table 3. The logistic regression models**

| | | **Basic model** | | | **Adjusted model** | | |
|---|---|---|---|---|---|---|---|
| | | **OR** | **95% CI** | ***P* value^{a}** | **OR** | **95% CI** | ***P* value^{b}** |
| **B2M/SAP MALDI ratio** | | | | | | | |
| | 0 | Ref. | Ref. | | Ref. | Ref. | |
| | >0 | 1.38 | 0.66-2.89 | 0.390 | 2.01 | 0.90-4.52 | 0.090 |
| **CC16/SAP MALDI ratio** | | | | | | | |
| | 0 | Ref. | Ref. | | Ref. | Ref. | |
| | >0 | 2.67 | 1.07-6.62 | 0.035 | 4.87 | 1.77-13.44 | 0.002 |

| **Combine B2M/SAP_MALDI and CC16/SAP MALDI ratio** | | | | | | | |
|---|---|---|---|---|---|---|---|
| | Group 1 | Ref. | Ref. | | Ref. | Ref. | |
| | Group2 | 1.32 | 0.60-2.90 | 0.495 | 1.95 | 0.82-4.66 | 0.134 |
| | Group3 | 3.07 | 0.95-9.94 | 0.061 | 7.59 | 1.97-29.24 | 0.003 |

| | | | | | | | |
|---|---|---|---|---|---|---|---|
| ^{a} *P* value for logistic regression model, unadjusted ^{b} *P* value for logistic regression model adjusted for follow up duration Group 1: B2M/SAP _MALDI ratio = 0 and CC16/SAP MALDI ratio = 0 Group2: B2M/SAP_MALDI ratio = 0 and CC16/SAP MALDI ratio > 0, B2M/SAP_MALDI ratio > 0 and CC16/SAP MALDI ratio = 0 Group3: B2M/SAP_MALDI ratio > 0 and CC16/SAP MALDI ratio > 0 | | | | | | | |

### 3. Discussion and Conclusions

Recently, MALDI-TOF MS has successfully approved as an in vitro diagnostic device for routine bacterial identification in hospitals [16]. Therefore, disease markers detected by MALDI-TOF MS is getting practical for clinical use. Because salts in urine can interfere with the MALDI-TOF mass spectral signals, in this study, a C18 plate was used to rapidly remove salts from clinical samples and retain urinary proteins in, urine samples for MALDI-TOF MS analysis. Our results showed that CC16 was more highly expressed in the WDM-NP and DM-NP groups than in the healthy (p < 0.001) and DM-WNP groups (p < 0.001). However, in the ELISA results, CC16 expression was only slightly higher in the DM-NP group than in the DM-WNP group (p = 0.05). These results also indicate that MALDI-TOF MS can identify CC16 more specifically than ELISA assay.

B2M is a low-molecular-weight protein that is filtered by the glomerulus and degenerated in the proximal tubules [17]. Some studies have shown that urinary B2M increases in renal tubular injuries, suggesting urinary B2M to be an early diagnostic marker of tubular injury [18,19]. In type 2 diabetes, urinary B2M excretion has been associated with macrovascular disease [20] and nephropathy [1,21,22].

The 15.8-kDa CC16 (also known as CC10, uteroglobin, or urinary protein 1) is rapidly eliminated by glomerular filtration, and reabsorbed and catabolized in the renal proximal tubule cells. Dysfunction of the proximal tubule cells can cause diminished resorption of CC16 and its increased levels in urine. CC16 has been reported as a marker of proximal tubular dysfunction in adult [23] and child patients [24]. This protein marker is sensitive to very subtle defects in proximal tubular dysfunction that may not be detected by assay of classical urinary low-molecular-weight proteins [25]. To the best of our knowledge, our study is the first to find high CC16 expression in urine of patients with nephropathy and DN.

With a long term follow-up study, the B2M and CC16 (OR of 7.59 for developing ERFD) were found to be independent predictors for ERFD among T2D patients who had not yet manifested significant kidney disease at baseline and indicated that the protein peaks of B2M and CC16 detected by C18 plate/MALDI-TOF may improve the sensitivity for predicting nephropathy before the appearance of urinary albumin.

From a large-sample size analysis, we discovered and validated 2 protein peaks, B2M (11.7 kDa) and CC16 (15.8 kDa), as biomarkers associated with nephropathy and verified the discriminatory ability in a set of 238 individuals including diabetic and nondiabetic patients. The OR of combined B2M and CC16 markers for developing ERFD was 7.59 (95% CI: 1.97-29.24). This is the first report of CC16 as a urinary marker of nephropathy and DN. Our approach of detecting B2M and CC16 by C₁₈ plate-MALDI-TOF MS may thus provide rapid diagnosis and prediction of nephropathy in type 2 diabetes patients.

### References

1. Dihazi H, Muller GA, Lindner S, Meyer M, Asif AR, et al. (2007) Characterization of diabetic nephropathy by urinary proteomic analysis: identification of a processed ubiquitin form as a differentially excreted protein in diabetic nephropathy patients. Clin Chem 53: 1636-1645.
2. Remuzzi G, Schieppati A, Ruggenenti P (2002) Clinical practice. Nephropathy in patients with type 2 diabetes. N Engl J Med 346: 1145-1151.
3. Otu HH, Can H, Spentzos D, Nelson RG, Hanson RL, et al. (2007) Prediction of diabetic nephropathy using urine proteomic profiling 10 years prior to development of nephropathy. Diabetes Care 30: 638-643.
4. Gerstein HC, Mann JF, Yi Q, Zinman B, Dinneen SF, et al. (2001) Albuminuria and risk of cardiovascular events, death, and heart failure in diabetic and nondiabetic individuals. JAMA 286: 421-426.
5. Indovina P, Marcelli E, Pentimalli F, Tanganelli P, Tarro G, et al. (2013) Mass spectrometry-based proteomics: the road to lung cancer biomarker discovery. Mass Spectrom Rev 32: 129-142.
6. Tan HT, Lee YH, Chung MC (2012) Cancer proteomics. Mass Spectrom Rev 31: 583-605.
7. Cravatt BF, Simon GM, Yates JR, 3rd (2007) The biological impact of mass-spectrometry-based proteomics. Nature 450: 991-1000.
8. Chang CT, Yang CY, Tsai FJ, Lin SY, Chen CJ (2015) Mass Spectrometry-Based Proteomic Study Makes High-Density Lipoprotein a Biomarker for Atherosclerotic Vascular Disease. Biomed Res Int 2015: 164846.
9. Papale M, Di Paolo S, Magistroni R, Lamacchia O, Di Palma AM, et al. (2010) Urine proteome analysis may allow noninvasive differential diagnosis of diabetic nephropathy. Diabetes Care 33: 2409-2415.
10. Wu J, Chen YD, Yu JK, Shi XL, Gu W (2011) Analysis of urinary proteomic patterns for type 2 diabetic nephropathy by ProteinChip. Diabetes Res Clin Pract 91: 213-219.
11. Liao HY, Tsai FJ, Lai CC, Tseng MC, Hsu CY, et al. (2016) Rapid fabrication of functionalized plates for peptides, glycopeptides and protein purification and mass spectrometry analysis. Analyst 141: 2183-2190.
12. Lindeman RD, Tobin J, Shock NW (1985) Longitudinal studies on the rate of decline in renal function with age. J Am Geriatr Soc 33: 278-285.
13. Levey AS, Bosch JP, Lewis JB, Greene T, Rogers N, et al. (1999) A more accurate method to estimate glomerular filtration rate from serum creatinine: a new prediction equation. Modification of Diet in Renal Disease Study Group. Ann Intern Med 130: 461-470.
14. Zhang Y, Oetting WS, Harvey SB, Stone MD, Monkkonen T, et al. (2009) Urinary Peptide patterns in native kidneys and kidney allografts. Transplantation 87: 1807-1813.
15. Chang CT, Liao HY, Huang WH, Lin SY, Tsai TY, et al. (2015) Early prediction of severe acute pancreatitis by urinary beta-2 microglobulin/saposin B peak ratios on MALDI-TOF. Clin Chim Acta 440: 115-122.
16. Seng P, Drancourt M, Gouriet F, La Scola B, Fournier PE, et al. (2009) Ongoing revolution in bacteriology: routine identification of bacteria by matrix-assisted laser desorption ionization time-of-flight mass spectrometry. Clin Infect Dis 49: 543-551.
17. Moresco RN, Sangoi MB, De Carvalho JA, Tatsch E, Bochi GV (2013) Diabetic nephropathy: traditional to proteomic markers. Clin Chim Acta 421: 17-30.
18. Sharifiaghdas F, Kashi AH, Eshratkhah R (2011) Evaluating percutaneous nephrolithotomy-induced kidney damage by measuring urinary concentrations of beta2-microglobulin. Urol J 8: 277-282.
19. Piscator M (1991) Early detection of tubular dysfunction. Kidney Int Suppl 34: S15-17.
20. Yoshikawa R, Wada J, Seiki K, Matsuoka T, Miyamoto S, et al. (2007) Urinary PGDS levels are associated with vascular injury in type 2 diabetes patients. Diabetes Res Clin Pract 76: 358-367.
21. Schardijn GH, Statius van Eps LW (1987) Beta 2-microglobulin: its significance in the evaluation of renal function. Kidney Int 32: 635-641.
22. Hong CY, Chia KS (1998) Markers of diabetic nephropathy. J Diabetes Complications 12: 43-60.
23. Bernard A, Lauwerys R, Noel A, Vandeleene B, Lambert A (1991) Determination by Latex Immunoassay of Protein-1 in Normal and Pathological Urine. Clinica Chimica Acta 201: 231-245.
24. Martin-Granado A, Vazquez-Moncholi C, Luis-Yanes MI, Lopez-Mendez M, Garcia-Nieto V (2009) Determination of Clara cell protein urinary elimination as a marker of tubular dysfunction. Pediatric Nephrology 24: 747-752.
25. Bernard A, Lauwerys R (1995) Low-Molecular-Weight Proteins as Markers of Organ Toxicity with Special Reference to Clara Cell Protein. Toxicology Letters 77: 145-151.
26. Kabanda A, Jadoul M, Lauwerys R, Bernard A, van Ypersele de Strihou C (1995) Low molecular weight proteinuria in Chinese herbs nephropathy. Kidney International 48(5): 1571-1576.

## Claims

1. A method for predicting diabetic nephropathy or early progressive renal function decline (ERFD) in a diabetic patient, the method comprising:
(i) providing a biological sample obtained from the diabetic patient; and
(ii) detecting a biomarker in the biological sample to obtain a detection level, comparing the detection level with a reference level for said biomarker to obtain a comparison result, and assessing whether the subject is at risk of developing nephropathy or ERFD based on the comparison result, wherein the biomarker includes Clara-cell protein (CC16) and an increase in the detection level as compared to the reference level is indicative of a higher risk of developing diabetic nephropathy or ERFD
wherein the biological sample is a urine sample.

2. The method of claim 1, wherein the detection is carried out by mass spectrometry or an immunoassay.

3. The method of claim 1,
wherein the biomarker includes CC16 and B2M.

## Patentansprüche

1. Verfahren zur Vorhersage von diabetischer Nephropathie oder frühzeitiger progressiver Nierenfunktionsverschlechterung (ERFD) bei einem diabetischen Patienten, wobei das Verfahren umfasst:
(i) Bereitstellen einer biologischen Probe, die von dem diabetischen Patienten erhalten wurde; und
(ii) Nachweisen eines Biomarkers in der biologischen Probe, um einen Nachweispegel zu erhalten, Vergleichen des Nachweispegels mit einem Referenzpegel für den Biomarker, um ein Vergleichsergebnis zu erhalten, und Beurteilen, ob das Subjekt ein Risiko hat, eine Nephropathie oder ERFD zu entwickeln, basierend auf dem Vergleichsergebnis, wobei der Biomarker das Clara-Zell-Protein (CC16) umfasst und ein Anstieg des Nachweispegels im Vergleich zum Referenzpegel ein höheres Risiko anzeigt, eine diabetische Nephropathie oder ERFD zu entwickeln;
worin die biologische Probe eine Urinprobe ist.

2. Verfahren nach Anspruch 1, wobei der Nachweis durch Massenspektrometrie oder einen Immunoassay durchgeführt wird.

3. Verfahren nach Anspruch 1, worin der Biomarker CC16 und B2M umfasst.

## Revendications

1. Méthode de prédiction de la néphropathie diabétique ou du déclin progressif précoce de la fonction rénale (ERFD) chez un patient diabétique, la méthode comprenant:
(i) fournir un échantillon biologique obtenu à partir du patient diabétique; et
(ii) détecter un biomarqueur dans l'échantillon biologique pour obtenir un niveau de détection, comparer le niveau de détection à un niveau de référence pour ledit biomarqueur afin d'obtenir un résultat de comparaison, et évaluer si le sujet est à risque de développer une néphropathie ou une ERFD sur la base du résultat de comparaison, dans lequel le biomarqueur comprend la protéine de la cellule de Clara (CC16) et une augmentation du niveau de détection par rapport au niveau de référence indique un risque plus élevé de développer une néphropathie diabétique ou une ERFD;
dans lequel l'échantillon biologique est un échantillon d'urine.

2. Méthode de la revendication 1, dans laquelle la détection est effectuée par spectrométrie de masse ou par un essai immunologique.

3. Méthode de la revendication 1, dans laquelle le biomarqueur comprend CC16 et B2M.
